(19) Europäisches Patentamt
European Patent Office
Office européen des brevets

(11) **EP 4 076 412 B1**

(12) **EUROPEAN PATENT SPECIFICATION**

(45) Date of publication and mention
of the grant of the patent:
**30.10.2024 Bulletin 2024/44**

(21) Application number: **20821418.9**

(22) Date of filing: **09.12.2020**

(51) International Patent Classification (IPC):
*A61K 31/015* (2006.01)   *A61K 33/242* (2019.01)
*A61K 31/28* (2006.01)   *A61K 31/336* (2006.01)
*A61P 13/00* (2006.01)   *A61P 15/00* (2006.01)
*A61P 31/00* (2006.01)   *A61P 31/04* (2006.01)
*A61P 31/10* (2006.01)

(52) Cooperative Patent Classification (CPC):
(C-Sets available)
**A61K 31/28; A61K 31/015; A61K 31/336;
A61K 33/242; A61P 13/00; A61P 15/00;
A61P 31/00; A61P 31/04; A61P 31/10**   (Cont.)

(86) International application number:
**PCT/IB2020/061676**

(87) International publication number:
**WO 2021/124023 (24.06.2021 Gazette 2021/25)**

(54) **GOLD- AND GRAPHENE-BASED MIXTURE AND COMPOSITIONS AND USE THEREOF**

MISCHUNG UND ZUSAMMENSETZUNGEN AUF GOLD- UND GRAPHENBASIS UND IHRE VERWENDUNG

MÉLANGE À BASE D'OR ET DE GRAPHÈNE ET COMPOSITIONS ET UTILISATION DE CELUI-CI

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **16.12.2019 IT 201900024132**

(43) Date of publication of application:
**26.10.2022 Bulletin 2022/43**

(73) Proprietor: **IDI Integratori Dietetici Italiani S.r.l.
95020 Aci Bonaccorsi (CT) (IT)**

(72) Inventor: **BOTTINO, Alessandro
95020 Aci Bonaccorsi (CT) (IT)**

(74) Representative: **Predazzi, Valentina et al
Società Italiana Brevetti S.p.A.
Piazza di Pietra, 39
00186 Roma (IT)**

(56) References cited:
**EP-A1- 2 130 531   CN-A- 107 754 004**

- **KOSTIANTYN TURCHENIUK ET AL: "Plasmonic photothermal destruction of uropathogenic E. coli with reduced graphene oxide and core/shell nanocomposites of gold nanorods/reduced graphene oxide", JOURNAL OF MATERIALS CHEMISTRY B, vol. 3, no. 3, 19 November 2014 (2014-11-19), GB, pages 375 - 386, XP055295487, ISSN: 2050-750X, DOI: 10.1039/C4TB01760A**
- **KOSTIANTYN TURCHENIUK ET AL: "Gold-graphene nanocomposites for sensing and biomedical applications", JOURNAL OF MATERIALS CHEMISTRY B, vol. 3, no. 21, 1 January 2015 (2015-01-01), GB, pages 4301 - 4324, XP055717308, ISSN: 2050-750X, DOI: 10.1039/C5TB00511F**

- **KAIYUAN ZHENG ET AL: "Synergistic Antimicrobial Capability of Magnetically Oriented Graphene Oxide Conjugated with Gold Nanoclusters", ADVANCED FUNCTIONAL MATERIALS, vol. 29, no. 46, 9 September 2019 (2019-09-09), DE, pages 1904603, XP055717307, ISSN: 1616-301X, DOI: 10.1002/adfm.201904603**

(52) Cooperative Patent Classification (CPC): (Cont.)

C-Sets
**A61K 31/015, A61K 2300/00;
A61K 31/336, A61K 2300/00;
A61K 33/242, A61K 2300/00**

## Description

The present invention relates to a mixture comprising gold particles and graphene particles or a derivative thereof, compositions comprising said mixture and use thereof in the treatment and/or prevention of urogenital tract infections.

## PRIOR ART

The use of molecules having antibacterial and/or antifungal activity for the treatment of infections in the urological-gynecological field is commonly associated with side and secondary effects that, very often, derive from a nontargeted action of the active molecules or from a systemic absorption thereof, even when administered with formulations for topical or external use, i.e. applied directly on the site of interest. Micro- and/or nanomedicine can provide the solution to this problem, due to the fact that human skin and mucosae commonly resist small-particle permeation, eliminating the concern regarding systemic absorption of the active molecules, if those are bound to nanoparticle carriers. It is therefore possible to bind an active molecule to a nanoparticle carrier modifying the molecule directing or absorption. Another strategy is that of using active agents that be themselves particles which, being unable to permeate skin barriers and mucosae, will carry out their pharmacological action directly and exclusively on the administration site, if suitably formulated, eliminating the problem of systemic side effects.

The use of nano- and microcapsules (NPs) of different nature, metallic, polymeric, lipidic, carbon-based, ceramic ones, etc., finds wide use in a biological and pharmaceutical field, such molecules can, e.g., be used as carriers to which one or more active molecules may be bound, or also for their inherent activities at a pharmaceutical level.

K Turcheniuk ET AL: "Plasmonic photothermal destruction of uropathogenic E.coli with reduced graphene oxide and core/shell nanocomposites of gold nanorods/reduced graphene oxide" JOURNAL OF MATERIALS CHEMISTRY B, vol. 3, no. 3, 19 November 2014 (2014-11-19), pages 375-386, discloses gold nanoparticles coated with pegylated graphene oxide for the selective killing of uropathogenic E. coli that be used in the treatment of patients with urinary tract infections.

K Turcheniuk ET AL: "Gold-graphene nanocomposites for sensing and biomedical applications", JOURNAL OF MATERIALS CHEMISTRY B, vol. 3, no. 21, 1 January 2015 (2015-01-01), pages 4301-4324, discloses various gold-graphene nanocomposites and their use for the photothermal ablation of bacteria. More particularly, gold nanoparticles coated with pegylated graphene oxide nanoparticles have been used tor the selective killing of uropathogenic E. coli and described as potential treatment of patients with urinary infections.

In the gynecological and urological field, the treatment of infections is, as mentioned above, carried out by the use of active molecules that are normally absorbed by the organism and have undesired side effects.

An example of drugs commonly used for the treatment of said infections is represented by antibiotics or by formulations containing them, or even by mixtures of plant essential oils such as, e.g., mint oil and others, which have a powerful antibacterial effect.

Such active principles, however, have undesired secondary effects like many drugs, as they also penetrate at a systemic level notwithstanding the topical application thereof. To date, no formulations for the topical treatment of urogenital infections exist which do not exhibit the above-reported technical problem.

## SUMMARY OF THE INVENTION

The Authors of the present invention solve the technical problem linked to current formulations for topical use for the treatment of urogenital infections, using, as active principles, gold and graphene micro- or nanoparticles or derivatives thereof or mixtures thereof in suitable mixtures or compositions, using a particular mixture of components having an antimicrobial effect markedly improved compared to that observed with the individual components. Moreover, the components of the mixture are not known to have secondary effects following topical administration, not even when administered systemically. Surprisingly, the Authors of the present invention have found that the combination of gold particles with graphene particles or derivatives thereof or mixtures thereof carries out a greater bactericidal and antifungal activity compared to that carried out by the individual components, with a relevant synergistic effect (see Examples 3 and 4). Moreover, as mentioned above, the use of particles of gold and of graphene (or a derivative thereof) of micro/nano sizes determines the absence/reduction of systemic absorption of the particles themselves, resulting in a localized therapeutic action limited to the site that is to be treated, i.e. the urogenital district.

Therefore, objects of the present invention are:

a mixture comprising:

- gold particles, and

- graphene particles or a derivative thereof or mixtures thereof,

and a linker binding said gold particles to said graphene particles or derivative thereof or mixtures thereof, wherein said linker is alpha lipoic acid for use in the treatment and/or prevention of urogenital tract infections;

wherein said derivative is graphene oxide.

a mixture comprising:

- gold particles,
- graphene oxide particles, and
- lactic acid

and a linker binding said gold particles to said graphene oxide particles wherein said linker is alpha lipoic acid

wherein said mixture has a pH of between 4 and 5, or wherein said mixture has pH 4.5;
a composition comprising such mixtures,
said composition for use in the treatment and/or prevention of urogenital tract infections and a medical device or kit comprising such mixture or such composition, processes for preparing said mixture or composition, the use of such mixtures, compositions, medical devices or kits for the treatment of male and female urogenital tract infections as defined in the present description.

**GLOSSARY**

In the present description, the terms "synergistic effect" or "synergistic activity" can be used indistinctly, as interchangeable; in both cases, in fact, an effect provided by the combination of two components that is greater than the sum of the individual effects of the individual components is denoted.
In the present description the term "dispersion", "suspension" and "solution" can be used indistinctly, as interchangeable at least in the micro- and nanometric sizes.
In the present description, "about" refers to the experimental error that may occur during conventional measurements. More particularly, when referring to a value it indicates $\pm$ 5% of the indicated value, and, when referred to a range, $\pm$ 5% of the ends thereof.
In the present description the term "topical use" denotes an external or an internal non-systemic use. Such use, therefore, also comprises an internal use by means of suppositories or creams with applicator or ovules or other medical devices, in which the active principles are intended for internal mucosae.

**DETAILED DESCRIPTION OF THE FIGURES**

Figure 1 shows the graph obtained by analyzing, through Dynamic Light Scattering (DLS), the sample containing the gold particles as indicated in the examples below, dispersed in citrate buffer (Aldrich cod. 741965) (DLS).

Figure 2 shows the graph obtained by analyzing, through DLS, the sample containing the graphene oxide particles as indicated in the examples below, dispersed in water (Aldrich cod. 795354).

Figure 3 shows the homogeneous dispersion of gold and graphene oxide particles, as reported in the examples below, in an aqueous phase mixture at pH 4.5. The acid pH was obtained by adding lactic acid to the starting aqueous phase.

Figure 4 shows the graph obtained by analyzing, through DLS, the sample containing the dispersion of gold and graphene oxide particles, as reported in the examples below, in mixture with an aqueous phase with pH= 4.5.

Figure 5 shows the graph obtained by analyzing, through DLS, the sample containing the dispersion of gold and graphene oxide particles, as reported in the examples below, in mixture in the presence of alpha lipoic acid in an aqueous phase with pH= 4.5. (*sample filtered with 0.5 micrometer filters prior to DLS analysis to eliminate any impurity interferences.)

Figure 6 shows the theoretical composition of the complex that can form among gold particles, graphene particles and alpha lipoic acid. The stoichiometric ratio seems in fact to be of ABOUT 1:1:1 (see Examples section).

## DETAILED DESCRIPTION

As mentioned above, one object of the present invention is represented by a mixture comprising:

- gold particles, and
- graphene particles or a derivative thereof or mixtures thereof,

and a linker binding said gold particles to said graphene particles or derivative thereof or mixtures thereof, wherein said linker is alpha lipoic acid,
for use in the treatment and/or prevention of urogenital tract infections,

wherein the graphene derivative is graphene oxide.

In one preferred embodiment of the invention, the mixture as described above and as claimed will further comprise an aqueous phase with an acid pH, said phase comprising at least one organic acid.
In particular, the pH of the mixture, in any one of the embodiments indicated above and below, could be from 3.5 to 7 or from 3.5 to 5 or, in a preferred embodiment, said pH could be of about 4,5.
The pH as indicated above and claimed can be obtained by addition to the mixture of the at least one organic acid indicated above, or of an inorganic acid such as hydrochloric acid. Such organic acid could be any one organic acid with from one to eight carbon atoms. Such acid could be one or more among methanoic, acetic, ethanoic, ethanedioic, oxoethanoic, 2-hydroxyethanoic, propanoic, prop-2-enoic, 2-propionic, propanedioic, 2-hydroxypropanedioic, oxopropanedioic, 2,2-dihydroxypropanedioic, 2-oxopropanoic, 2-hydroxypropanoic (lactic acid), 3-hydroxypropanoic, 2,3-dihydroxypropanoic, butanoic, 2-methylpropanoic, 2-oxobutanoic, 3-oxobutanoic, 4-oxobutanoic, (E)-butenedioic, (Z)-butenedioic, but-2-enedioic, oxobutanedioic, hydroxybutanedioic, 2,3-dihydroxybutanedioic, (E)-but-2-enoic, pentanoic, 3-methylbutanoic, pentanedioic, 3-oxopentanedioic, hexanoic, hexanedioic, 2-hydroxypropane-1,2,3-tricarboxylic, prop-1-en-1,2,3 tricarboxylic, 1-hydroxypropane-1,2,3 tricarboxylic, (2E,4E)-hexa-2,4-dienoic, heptanoic, cyclohexanecarboxylic, benzenecarboxylic, 2-hydroxybenzoic, octanoic, benzene-1,2-dicarboxylic acid.
In one preferred embodiment, said acid will be 2-hydroxypropanoic acid, better known as lactic acid.
According to the present invention, said lactic acid could be in any enantiomeric form or a racemate thereof, therefore the mixture could comprise S-(+)-lactic acid, L-(+)-lactic acid S-(-)-lactic acid, L-(-)-lactic acid, or mixtures thereof. In one embodiment the mixture comprises L-(+)-lactic acid and/or L-(-)-lactic acid.
According to the present invention, the mixture for the use described above and claimed, said gold particles can have sizes from 1 nm to 2 $\mu$m, or from 20 nm to 1 $\mu$m and said graphene particles or derivative thereof can have sizes from 1 nm to 2 $\mu$m, or from 20 nm to 1 $\mu$m. According to the invention, any combination of the particles as defined above is suitable.
Therefore, the mixture could contain gold particles of sizes from 1 nm to 2 $\mu$m in combination with graphene particles or derivative thereof of sizes from 1 nm to 2 $\mu$m or mixtures thereof; gold particles of sizes from 20 nm to 1 $\mu$m in combination with graphene particles or derivative thereof of sizes from 1 nm to 2 $\mu$m or mixtures thereof, graphene particles or derivative thereof of sizes from 20 nm to 1 $\mu$m in combination with gold particles of sizes from 1 nm to 2 $\mu$m or mixtures thereof.
In one embodiment of the invention mixtures are preferred in which at least one type of particles has colloidal sizes (from 10 to 1000 nm) or in which both types of particles have sizes from 20 nm to 1 $\mu$m.
The mixture comprises a linker as defined below and the particles of gold and graphene or derivative thereof form a complex, in the examples defined as "gold/graphene complex". The mixture of the invention, therefore, comprises the gold-linker-graphene particle complex or derivative thereof.
Said complex will preferably have sizes in the above-indicated ranges for the individual particles.
According to the present invention, the mixture could comprise gold particles and graphene particles (or derivative thereof or mixtures thereof) in different weight ratios. By way of a non-limiting example, the weight ratio between said gold particles and said graphene particles or derivative thereof or mixtures thereof could be from 1:1 a 1:100, preferably from 1:1 to 1:40 or more preferably of 1:1; or, the weight ratio between said graphene particles or derivative thereof or mixtures thereof and said gold particles could be from 1:1 to 1:100 or, preferably, from 1:1 to 1:40. In one preferred embodiment, the weight ratio between the aforesaid particles is of about 1:1.
In particular, in any embodiment disclosed in the present description, the concentration of said gold particles could be, e.g., from 0.00001% to 0.2% w/w, preferably from 0.00001 to 0.1% w/w and more preferably from 0.00001 to 0.004% w/w and the concentration of said graphene particles or derivative thereof or mixtures thereof could be from 0.00001% to 0.2% w/w, preferably from 0.00001 to 0.1% w/w or, more preferably, from 0.00001 to 0.004% w/w.

In one embodiment the concentration of said gold particles can be from 0.0001 to 0.004% w/w, preferably from 0.001 to 0.1% w/w, and the concentration of said graphene oxide particles can be from 0.0001 to 0.004% w/w or from 0.001 to 0.1% w/w.

According to the present invention, the mixture, in any one of the embodiments provided in the present description or claimed, comprises a linker binding said gold particles to said graphene particles or derivative thereof or mixtures thereof, wherein said linker is alpha lipoic acid. Examples of suitable linkers that are not part of the invention is represented by a natural or synthetic molecule comprising one or more portions capable of interacting with at least one gold particle, and one or more portions capable of interacting with at least one graphene particle or derivative thereof e.g. comprising, in said portion capable of interacting with at least one gold particle, one or more sulfur and/or nitrogen atoms and, in said portion capable of interacting with at least one graphene particle or derivative thereof, one or more chemical groups capable of forming hydrophobic interactions and/or hydrogen bonds.

Chemical groups capable of forming hydrogen bonds comprises chemical groups selected from carboxylic acids, esters thereof, amino groups and/or derivatives thereof (such as alkyl amines) and alcohol groups.

According to the invention, said linker is alpha lipoic acid

According to the present invention, in one embodiment applicable to any of the above-described embodiments, said alpha lipoic acid could have a concentration ranging from 0.0000001 to 0.1% w/w, preferably from 0.0000001 to 0.00001% w/w.

A further object of the invention is represented by a mixture, as such and not limited to the use, comprising:

- gold particles,
- graphene oxide particles, and
- lactic acid

and a linker binding said gold particles to said graphene oxide particles wherein said linker is alpha lipoic acid wherein said mixture has a pH of between 3.5 and 7, or between 4 and 6, or wherein said mixture has a pH of about 4.5.

In the present description, the mixture as a product not limited to therapeutic use for the treatment and/or the prevention of urogenital tract infections is also simply referred to as "mixture as such" in order to distinguish it from the mixture for therapeutic use described above and object of the claims.

In one embodiment of the above-described mixture as such, said gold particles can have sizes from 1 nm to 2 $\mu$m, preferably from 20 nm to 1 $\mu$m, and said graphene oxide particles can have sizes from 1 nm to 2 $\mu$m, preferably from 20 nm to 1 $\mu$m.

In the mixture as such object of the invention, the weight ratio between said gold particles and said graphene oxide particles is from 1:1 to 1:40, preferably of 1:1; or the weight ratio between said graphene oxide particles and said gold particles is from 1:1 to 1:40.

In the mixture as such object of the invention, in any embodiment provided in the present description, wherein the concentration of said gold particles is from 0.00001% to 0.2% w/w, or from 0.00001 to 0.1% w/w, or from 0.00001 to 0.004% w/w, or from 0.0001 to 0.004% w/w, or from 0.001 to 0.1% w/w and the concentration of said graphene oxide particles, is from 0.00001% to 0.2% w/w, or from 0.00001 to 0.1% w/w and/or from 0.00001 to 0.004% w/w, or from 0.0001 to 0.004% w/w or from 0.001 to 0.1% w/w.

In one embodiment the concentration of said gold particles can be from 0.0001 to 0.004% w/w, preferably from 0.001 to 0.1% w/w and the concentration of said graphene oxide particles can be from 0.0001 to 0.004% w/w or from 0.001 to 0.1% w/w.

Also the mixture as such as defined herein, in any one of the embodiments provided, comprises a linker binding said gold particles to said graphene oxide particles, wherein said linker is alpha lipoic acid.

Preferably, said alpha lipoic acid will have a concentration ranging from 0.0000001 to 0.00001, or also a concentration from 0.00002 to 0.0001% w/w.

Non-limiting examples of said mixture are provided hereinafter.

[Having shifted from parts in weight to grams]

Example 1

mixture comprising, per 100 grams of final formulation:

0.001 g of gold particles,
0.001 g of graphene oxide particles,
0.9 g of lactic acid, and

0.00001 g of alpha lipoic acid.

Object of the invention is also a pharmaceutical composition comprising the mixture (for the use described in the treatment and/or the prevention of urogenital tract infections, or as such), and at least one pharmaceutically acceptable excipient.

The technician in the field will know how to select said excipient according to the type of formulation to be made, and other further additives, on the basis of his/her general knowledge of the pharmacopoeia.

According to some embodiments, said composition could be made in liquid, solid or semi-solid form, in the form of cream, gel, cream-gel, emulgel, softgel, foam, emulsion, sprayable emulsion, suppository.

One example of pharmaceutical cream composition is provided hereinafter.

Another formulation example also object of the invention, is provided in the

"Example" section below.

Composition example 1:

an O/W cream consisting of:

0.001 g of gold particles
0.001 g of graphene oxide particles
0.00001 g of alpha lipoic acid
0.5 g of lactic acid
30 g of liquid paraffin
10 g of cetearyl alcohol
4 g of PEG 1500
1g of Tween 80
Water q.s. to 100 g.

Object of the invention is also the mixture as such according to any one of the embodiments provided, for use as a medicament, e.g. for use in the treatment and/or prevention of urogenital tract infections.

Object of the invention is also the composition of the invention, e.g. for use in the treatment and/or prevention of urogenital tract infections.

According to the present invention, such infections may be bacterial and/or fungal and/or protozoan and/or mycoplasma infections.

In one embodiment of the invention said bacterial infections may be infections caused by gram-negative, gram-positive and/or gram-variable bacteria.

According to a further embodiment, said fungal infections may be infections caused by a fungus of the genus Candida.

The mixture or the composition of the invention are preferably meant for topical use, i.e. intended in particular for skin or mucosae (external or internal; both male and female ones).

In particular, said topical use can be carried out by applying said mixture or composition externally, internally, on the skin, on the mucosa, in the vagina, on the vulva or on the penis.

Object of the invention is the mixture or the composition according to any one of the embodiments of the invention for use for the prevention and/or the treatment of urogenital tract infections as defined above, by topical application (internal or external, as explained in the present description).

A further object of the invention is a mixture according to any of the embodiments described, for preparing a pharmaceutical composition or a medical device for use in the treatment and/or prevention of urogenital tract infections, as well as a process for preparing a pharmaceutical composition as described or claimed, wherein a mixture according to any of the embodiments described is additioned with at least one pharmaceutically acceptable excipient.

A further object of the invention are medical devices comprising the mixture or the composition according to the present description in any one of the embodiments indicated, or a kit for applying the aforesaid mixture or composition, said kit comprising aliquots of said mixture or composition introduced or to be introduced into suitable applicators, such as, e.g. vaginal applicators, single-dose sachets.

Finally, object of the invention are a vaginal capsule, vaginal tablet, vaginal softgel, ovum, vaginal films, bandage, plaster, wadding, spray, probe comprising the mixture or the composition according to the present description in any one of the embodiments indicated.

Anywhere in the present description the term comprising may be replaced by the term "consisting of" or "being consisting of".

The examples provided hereinafter illustrate in a non-limiting manner the invention and some embodiments thereof.

## EXAMPLES

In the following examples the following active principles, based on identified colloidal gold and graphene, were used:

- Gold nanoparticles, 20 nm diameter, OD 1, stabilized suspension in citrate buffer (Cod. 741965, 100 mL, Aldrich);
- Graphene oxide nanocolloids, 2 mg/mL, dispersion in $H_2O$
  (Cod. 795534, 200 mL, Aldrich).

The Inventors declare that all cell lines used are commercial cell lines, and that the provisions of the decrees cited in Art. 170bis c.4, specifically related to the confined use of genetically modified microorganisms, were complied with.

## EXAMPLE 1.

**Stabilization test of the graphene- and gold-based colloidal mixture and characterization thereof by dynamic light scattering (DLS) characterization** subdivided in the following sections:

1) Preliminary study on the commercial gold colloid;
2) Preparing and stabilizing of the gold/graphene complex and DLS analysis.

### 1) Preliminary study

A preliminary analysis was carried out on the colloidal gold sample provided by Sigma Aldrich Company (20nm Gold nanoparticles, Code 741965) in order to determine the amount of colloidal gold per volume unit of colloidal dispersion. For that purpose, the sample was subjected to UV-Vis analysis at a 400-nm wavelength, and absorbance was compared to that of a standard of known concentration. The analysis revealed that the sample of commercial colloidal gold has a composition of 0.05 mg/ml of colloidal gold. For the sample of commercial colloidal graphene (Graphene oxide nanocolloids 2mg/mL, cod. 795534) the weight/volume composition is provided by the manufacturer. The colloidal dispersions, both of gold and of graphene, were analyzed by DLS in order to confirm mean sizes, dispersity, and surface potential. The DLS graphs, reported hereinafter, confirmed the colloidal sizes and a good polidispersity index (see Table 1 and Graph 1 reported hereinafter). The mean hydrodynamic radius detected for the gold sample proved consistent with that declared by the manufacturer in the technical specifications (Mean Hydrodynamic Diameter (Z) 28-36 nm). At DLS, the mean sizes of the graphene particles proved, as expected, greater than those detected by the manufacturing company (nanocolloid= <100nm) owing to the high scattering due to the morphology of graphene itself (M. Lotya et al. Nanotechnology 24 (2013) 265703)*. However, this feature proved to be advantageous for this study, as it allowed to effectively discriminate the two colloid populations. The graphene polydispersity index proved to be very good:

*Table 1*

| Sample | Size (nm ± D.S.) | Zeta potential | PDI |
|---|---|---|---|
| Gold nanoparticles | 34 ± 13 | -20.3 | 0.19 |
| Graphene oxide | 561* ± 110 | -21.5 | 0.28 |
| Gold/graphene mixture, pH 4.5 | 616* ± 251<br>35 ± 10 | -15.6 | 0.82 |
| Gold/graphene complex, pH 4.5 + lipoic acid | 776* ± 171<br>**88 ± 24**<br>30 ± 7 | -14.4 | 0.68 |

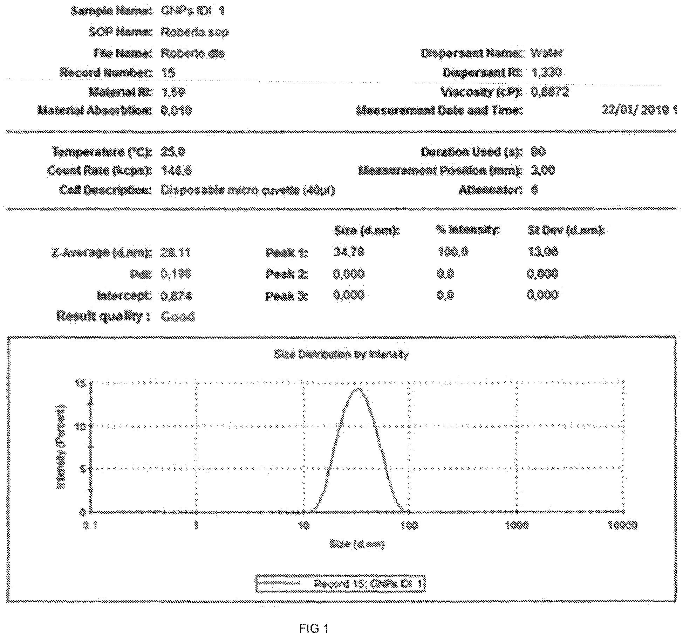

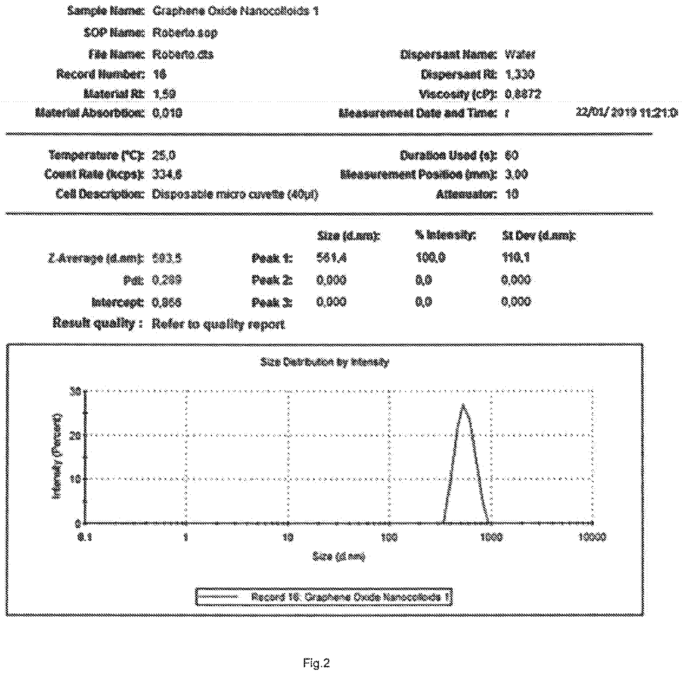

See also figure 1 and figure 2.

### 2) **Preparing and stabilizing the gold/graphene complex, and DLS analysis**

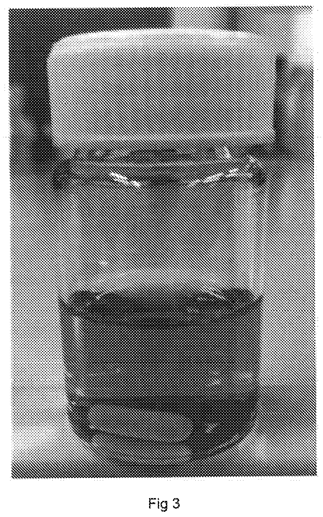

Exactly measured volumes of the colloidal dispersions of gold and graphene, respectively, were suitably mixed **so as to obtain a gold/graphene weight ratio of 1/1.** Specifically, 20 ml of gold colloidal dispersion (containing 1 mg of gold) was mixed with 0.5 ml of graphene colloidal dispersion (containing 1 mg of graphene). The obtained dispersion was acidified to a pH value of 4.5 using a 1% lactic acid aqueous solution. The final dispersion proved to be homogeneous and of a dark pink color (Figure 3).

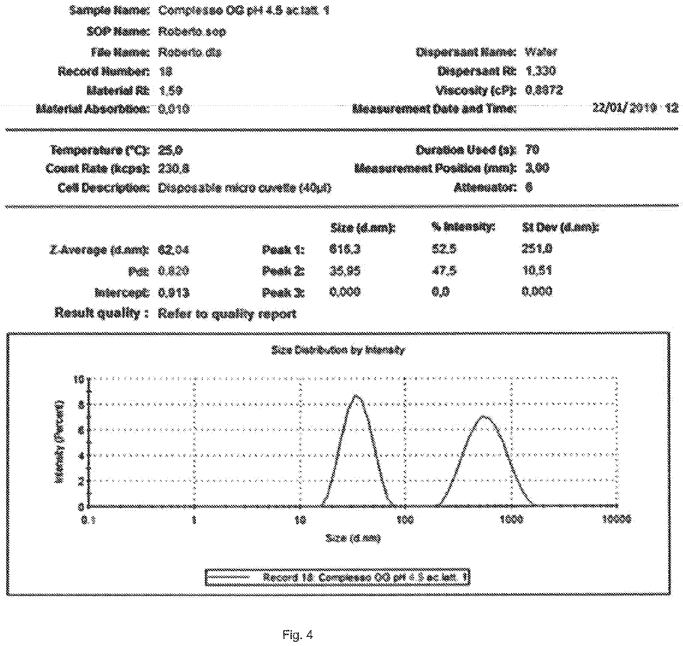

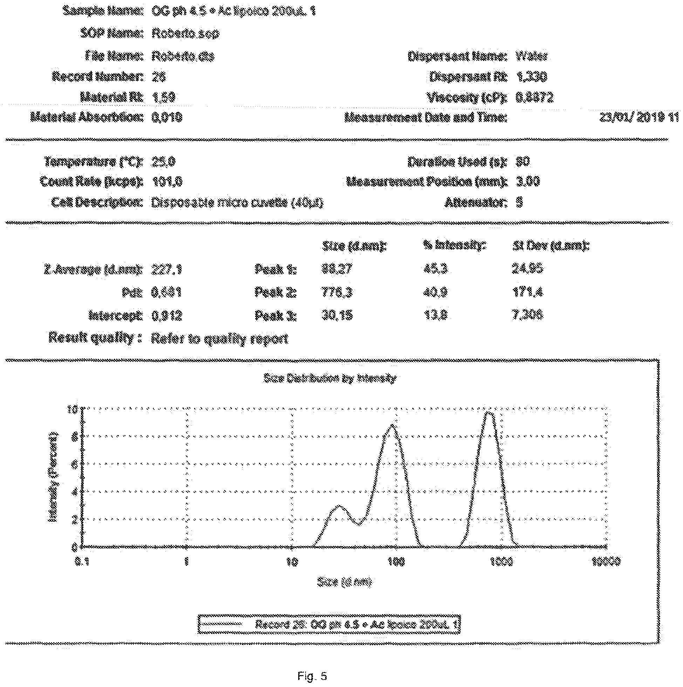

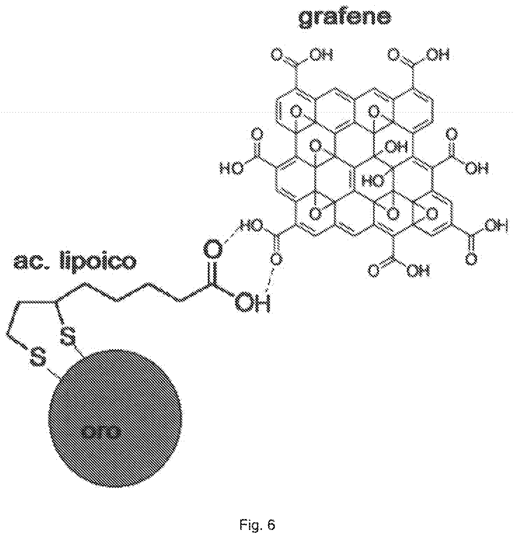

DLS analysis of the colloidal dispersion showed the presence of two distinct populations having sizes consistent with those of the gold particles and the graphene particles, respectively (Figure 4). Gold and graphene particle aggregation was induced by adding to the acidified colloidal mixture increasing aliquots of a saturated solution of alpha lipoic acid at pH 4.5 in bidistilled water. The formation of a stable gold/graphene complex was obtained by using a volume ranging between 0.1-0.2 ml of alpha lipoic acid solution, at the concentration of 0.1 mg/mL, in 20.5 ml of the starting colloidal mixture. Alpha lipoic acid binds to the surface of gold, generating hydrophobic interactions and hydrogen with the graphene. DLS analysis highlighted the appearance of a new population of particles (gold/graphene complex) with mean sizes of 88 nm and higher intensity (Figure 5), in equilibrium with the original colloidal species. The forming of the complex does not alter the color of the colloidal dispersion. However, the further addition of alpha lipoic acid solution destabilizes the system, generating the appearance of a dark precipitate. In confirmation of the gold/graphene complex forming, also aliquots of sole gold or of sole graphene were analyzed in the presence of alpha lipoic acid, without the occurrence of interaction phenomena. Therefore, without being bound by theory, it may be assumed that the 88-nm peak be actually comprised of a "near-stoichiometric" association of a gold particle with a graphene particle, as illustrated in Figure 6.

## EXAMPLE 2:

The colloidal gold- and graphene-based active principles used in the examples are the following:

- 20-nm diameter gold nanoparticles, OD 1, stabilized suspension in citrate buffer (Code 741965, 100 mL, Aldrich);

- Graphene oxide nanocolloids, 2 mg/mL, dispersion in $H_2O$
  (Cod. 795534, 200 mL, Aldrich).

**A. Oily formulation for vaginal softgel capsules** based on: colloidal gold (0.001-0.002% w/v) stabilized in oil with alpha lipoic acid or 1-dodecanethiol; colloidal graphene (0.001-002% w/v); oily phase caprylic/capric triglycerides; stabilizers, viscosifiers, emulsifiers (emulgators), preservatives and any other coformulants.

**B. Cream** based on colloidal gold (0.001-0.002% w/v) and colloidal graphene (0.001-0.002% w/v) of the W/O emulsion type. Cream based on colloidal gold (0.1-0.2% w/v) and colloidal graphene (0.001-0.002% w/v) of the O/W emulsion type. The oily phase could be consisting of caprylic/capric triglycerides, PH.EUR vaseline oil, white vaseline. The aqueous phase will contain water and polyethylene glycol, stabilizers, viscosifiers, emulsifiers, preservatives and any other coformulants.

## O/W CREAM Example B1: (Reference example)

**Oily phase:**

- 25 g liquid paraffin
- 10 g Cetearyl Alcohol
- 5 g caprylic/capric triglyceride

**Aqueous phase:**

- 50.9 g $H_2O$
- 5 g Ceteareth
- 2 g Gold nanoparticle solution (gold 0.05 mg/mL)
- 2 g Graphene oxide nanocolloid (graphene 2 mg/mL)
- 0.025 g Nipasol
- 0.075 g Nipagin

The exactly weighed amount of Ceteareth was solubilized in bidistilled water under stirring and in a water bath at 60°C. Upon solubilization, the gold and graphene solutions, and subsequently the solid-state antimicrobials were added. The exactly weighed amount of liquid paraffin and caprylic/capric triglyceride were put in a water bath at the temperature of 80 °C. Subsequently, Cetearyl alcohol was added until complete melting. The mixing of the two phases occurred by using an Ultraturrax VDI 25 (VWR): the aqueous phase was slowly added to the oily phase under the action of the machinery at 17.500 rpm for 10 min, and when the emulsion temperature had reached 50°C said emulsion was left to cool down under constant stirring at 450 rpm using a blade stirrer. At room temperature the cream is stable, has a homogeneous appearance, fluid consistency and white color.

**O/W CREAM example B2:** (Reference example)

**Oily phase:**

- 30 g Liquid paraffin
- 10 g Cetearyl alcohol

**Aqueous phase:**

- 50.9 g $H_2O$
- 4 g PEG
- 2 g Gold nanoparticle solution (gold 0.05 mg/mL)
- 2 g Graphene oxide nanocolloid (graphene 2 mg/mL)
- 1 g Tween 80
- 0.025 g Nipasol
- 0.075 g Nipagin

The exactly weighed amounts of Tween 80 and PEG were solubilized in bidistilled water under stirring and in a water bath at 60°C. Upon solubilization, the gold and graphene solutions, and subsequently the solid-state antimicrobials were added. The exactly weighed amounts of liquid paraffin and cetearyl alcohol were put in a water bath at the temperature of 80 °C until complete melting. The mixing of the two phases occurred by using an Ultraturrax VDI 25 (VWR): the aqueous phase was slowly added to the oily phase under the action of the machinery at 17.500 rpm for 10 min, and slight foaming was observed at this stage. When the emulsion temperature had reached 50°C said emulsion was left to cool down under constant stirring at 450 rpm using a blade stirrer. At room temperature the cream is stable, has a homogeneous appearance, fluid and soft consistency, white color.

**O/W CREAM example B3:** (Reference example)

**Oily phase:**

- 30 g Liquid paraffin
- 10 g Cetearyl alcohol

**Aqueous phase:**

- 34 g $H_2O$
- 20 g Aqueous gold nanoparticle solution (gold 0.05 mg/mL)
- 4 g PEG 1500
- 0.5 g Tween 80
- 0.5 g Graphene oxide nanocolloid in water (graphene 2 mg/mL)
- 0.9 g Lactic acid
- 0.025 g Nipasol
- 0.075 g Nipagin

The exactly weighed amounts of Tween 80 and PEG were solubilized in bidistilled water under stirring. Upon solubilization, the solid-state antimicrobials and the gold and graphene solutions were added, and lactic acid was added until obtaining a final pH of the aqueous phase equal to 4.5. Upon reaching a pH value equal to 5.5 the forming of a dark precipitate was observed, which increased with pH lowering.

In the subsequent tests the gold-graphene mixture was stabilized in an acid solution with alpha lipoic acid, and their concentrations were balanced in order to obtain a stable formulation, with required color and pH.

**O/W CREAM example B4:**

**Oily phase:**

- 30 g Liquid paraffin
- 10 g Cetearyl alcohol

**Aqueous phase:**

- 34 g $H_2O$
- 20 g Aqueous gold nanoparticle solution (gold 0.05 mg/mL)
- 4 g PEG 1500
- 1 g Tween 80
- 0.5 g Graphene oxide nanocolloid in water (graphene 2 mg/mL)
- 0.5 g Lactic acid
- 0.00001 g Alpha lipoic acid

The exactly weighed amounts of Tween 80 and PEG were solubilized in bidistilled water under stirring. Upon solubilization, the gold and graphene solutions were added and lactic acid was added until obtaining a final pH of the aqueous phase equal to 4.5. The exactly weighed amounts of liquid paraffin and cetearyl alcohol were put in a water bath at the temperature of 70 °C until complete melting. The mixing of the two phases occurred by using an Ultraturrax VDI 25 (VWR) and thermostating the process at 50 °C: the aqueous phase (previously heated at 50 °C) was slowly added to the oily phase under the action of the machinery at 17.500 rpm for 10 min. When the emulsion temperature reached 50°C said emulsion was let to cool down under constant stirring at 500 rpm using blade stirrer, thermostating the process at 30 °C. At room temperature the cream is stable, has a homogeneous appearance, fluid consistency, pink color.

**W/O CREAM example B5:**

**Aqueous phase:**

- 18 g $H_2O$
- 20 g Aqueous gold nanoparticle solution (gold 0.05 mg/mL)
- 0.5 g Graphene oxide nanocolloid in water (graphene 2 mg/mL)
- 0.025 g Nipasol
- 0.075 g Nipagin
- 1 g Phospholipon 80
- 0.5 g Lactic acid
- 0.00001 g Alpha lipoic acid

**Oily phase:**

- 39 g White vaseline

- 8 g Liquid paraffin
- 5 g Caprylic/capric triglyceride
- 5 g Soy lecithin
- 2 g Cetearyl alcohol
- 1 g Emulgator

The exactly weighed amount of Phospholipon 80H was solubilized in bidistilled water under stirring and in a water bath at 60°C. Upon solubilization, the gold and graphene solutions, and subsequently the solid-state antimicrobials were added. The exactly weighed amount of white vaseline was put in a water bath at the temperature of 80 °C. Subsequently, liquid paraffin, caprylic/capric triglyceride and cetearyl alcohol were added. Upon reaching the total melting of the components a homogeneous liquid phase was obtained and soy lecithin (the oily phase turns deep yellow) and the emulgator (emulsifier) were added

The mixing of the two phases occurred by using an Ultraturrax VDI 25 (VWR): the aqueous phase was slowly added to the oily phase under the action of the machinery at 17.500 rpm for 10 min, and when the emulsion temperature reached 50°C said emulsion was left to cool down under constant stirring at 450 rpm using a blade stirrer. At room temperature the cream is stable, has a homogeneous appearance, fluid consistency and yellow color.

## EXAMPLE 3:

Microbiological testing of the gold-graphene-lipoic acid mixture according to the invention, and of the superiority thereof compared to the single active agents (gold or graphene) individually, in an aqueous phase:

> Determining the MIC and MBC of the formulation containing the gold-graphene mixture with presumed antibacterial activity toward free-living ( planktonic) microbial strains of clinical interest in the uro-gynecological field: *Escherichia Coli, Enterococcus faecalis, Streptococcus agalactiae, Gardnerella vaginalis, Candida albicans, Candida krusey*
> Assessment of the activity inhibiting the forming of the biofilm by the above-indicated strains
> Assessment of the activity disrupting the 24h-mature biofilm formed by the above-indicated strains

The antibacterial and antifungal activity of the gold + graphene + lipoic acid (nanogold particles 0.05 mg/ml + graphene 0.05 mg/ml + lipoic acid 0.0005 mg/ml) aqueous mixture, prepared by mixing 20 ml of aqueous solution of nanogold particles 0.05 mg/Ml + 0.5 ml of graphene oxide nanocolloid in water (graphene 2 mg/mL) + 0.5 g of lactic acid + 0.1 ml of a 0.1 mg/ml alpha lipoic acid aqueous solution was assessed in terms of MIC (Minimum Inhibitory Concentration) in % v/v or in weight concentration (m/ml), by using a 96-well plate micromethod, said method briefly consists in a 1:2 serial dilution of the sample in the suitable culture medium (MH in case of bacteria or Sabouraud Broth in case of fungi), in the adding of the microbial inoculum, incubating at 37°C for 24h, reading the optical density (OD) of the wells, comparing between the OD of the sample wells and of the positive control wells (untreated growth control) and the negative control wells (sole culture medium and sample at the different concentrations, but without microbial inoculum) and finally in the determining of the MIC.

### 3.1 Activity of the gold-graphene-lipoic acid mixture toward *Streptococcus Agalactiae*

Table 2

| MIC (% v/v) | |
|---|---|
| | S. *Agalactiae ATCC 13813* |
| gold+graphene+lipoic acid mixture (0.05 mg/ml + 0.05 mg/ml + 0.0005 mg/ml) As reported above | 50 |
| Gold nanoparticles (0.05 mg/ml) | 50 |
| Graphene (2 mg/ml) | 50 |

Micromethod by dilution in Mueller Hinton liquid medium

The gold+graphene+lipoic acid mixture (gold nanoparticles 0.05 mg/ml + graphene 0.05 mg/ml + lipoic acid 0.0005 mg/ml), (Examples B4 and B5), exhibited an interesting antimicrobial activity against a free-living strain of S.

*Agalactiae* ATCC 13813.

The mixture tested in the bacterial inhibition studies reported below constitutes a part of the components of the aqueous phase of Examples B4 and B5, i.e.: 20 ml of aqueous solution of nanogold particles 0.05 mg/ml + 0.5 ml of nanocolloid graphene oxide in water (graphene 2 mg/mL) + 0.5 g of lactic acid + 0.1 ml of an aqueous solution of 0.1 mg/ml alpha lipoic acid.

Said mixture corresponds to (0.05 mg/ml nanogold particles + graphene 0.05 mg/ml + 0.0005 mg/ml lipoic acid). The lactic acid was used to bring the pH to 4.5.

The tested mixture constitutes a part of the components of the aqueous phase of Examples B4 and B5, i.e.: 20 ml of aqueous solution of nanogold particles 0.05 mg/ml + 0.5 ml of nanocolloid graphene oxide in water (graphene 2 mg/mL) + 0.5 g of lactic acid + 0.1 ml of a 0.1 mg/ml alpha lipoic acid aqueous solution.

## 3.2 Activity of the gold-graphene-lipoic acid mixture toward Escherichia Coli

Table 3

| MIC (% v/v) | |
| --- | --- |
| | *E. Coli ATCC 25922* |
| gold-graphene-lipoic acid mixture (0.05 mg/ml + 0.05 mg/ml + 0.0005 mg/ml) as indicated above | 3.1 |
| | |
| Gold nanoparticles (0.05 mg/ml) | 50 |
| Graphene (2 mg/ml) | 25 |

Micromethod by dilution in Mueller Hinton liquid medium

The gold-graphene-lipoic acid mixture (gold nanoparticles 0.05 mg/ml + graphene 0.05 mg/ml + lipoic acid 0.0005 mg/ml), (Examples B4 and B5), exhibited an interesting antimicrobial activity against a free-living strain of E. Coli ATCC 25922.

## 3.3 Activity of the gold-graphene mixture-lipoic acid mixture toward *Enterococcus faecalis*

Table 4

| MIC (% v/v) | |
| --- | --- |
| | *E. Faecalis ATCC 29212* |
| Gold-graphene-lipoic acid mixture (0.05 mg/ml + 0.05 mg/ml + 0.0005 mg/ml) as indicated above | 3.1 |
| Gold nanoparticles (0.05 mg/ml) | 50 |
| Graphene (2 mg/ml) | 25 |

Micromethod by dilution in Mueller Hinton liquid medium

The gold-graphene-lipoic acid mixture (gold nanoparticles 0.05 mg/ml + graphene 0.05 mg/ml + lipoic acid 0.0005 mg/ml), (Examples B4 and B5), exhibited an interesting antimicrobial activity against a free-living strain of *E. Faecalis* ATCC 29212.

## 3.4 Activity of the gold-graphene-lipoic acid mixture toward *Candida albicans* and *Candida krusei*

Table 5

| | MIC (% v/v) | |
|---|---|---|
| | C. *albicans* ATCC 10536 | C. *krusei* ATCC 14243 |
| Gold-graphene-lipoic acid mixture (0.05 mg/ml + 0.05 mg/ml + 0.0005 mg/ml) as indicated above | 12.5 | 50 |
| Gold nanoparticles (0.05mg/ml) | 50 | 50 |
| Graphene (2mg/ml) | 50 | 50 |

Micromethod by dilution in Sabouraud liquid medium

The gold-graphene-lipoic acid mixture (gold nanoparticles 0.05 mg/ml + graphene 0.05 mg/ml + lipoic acid 0.0005 mg/ml), (Examples B4 and B5), exhibited antifungal activity against free-living strains of C. *albicans* ATCC 10536 and C. *krusei* ATCC 14243.

The results of the microbiological tests obtained to date demonstrate the superiority of the gold-graphene-lipoic acid mixture (gold nanoparticles 0.05 mg/ml + graphene 0.05 mg/ml + lipoic acid 0.0005 mg/ml), aqueous phase of the final formulation in cream (Examples B4 and B5), compared to the single active agents (gold or graphene) at the same (gold) or higher (graphene) concentration.

The gold-graphene-lipoic acid formulation (gold nanoparticles 0.05 mg/ml + graphene 0.05 mg/ml + lipoic acid 0.0005 mg/ml (aqueous phase of the cream), in fact, has an interesting antimicrobial activity against reference E. *coli* ATCC 25922 and E. *faecalis* ATCC 29212 strains and has a MIC of 3.1% v/v against both strains, said MIC in % v/v corresponds to a MIC in concentration by weight equal to 1.5 $\mu$g/ml+1.5 $\mu$g/ml.

The MIC against a reference S. *agalactiae* ATCC 13813 strain is equal to 50% v/v, corresponding to a MIC by weight of 25 $\mu$g/ml+25 $\mu$g/ml.

The gold-graphene-lipoic acid mixture (gold nanoparticles 0.05 mg/ml + graphene 0.05 mg/ml + lipoic acid 0.0005 mg/ml, aqueous phase of the cream, has an interesting antifungal activity against C.*albicans* ATCC 10536 and exhibits a MIC equal to 12.5% v/v (6.2$\mu$g/ml+6.2$\mu$g/ml by weight). Toward C.*krusei* ATCC 14243 it exhibits a MIC of 50% v/v (25 $\mu$g/ml+25 $\mu$g/ml by weight).

In view of the important role in chronic and persistent infections and in the resistance to antibiotics of the complex microbial communities referred to as biofilm, the Inventors proceeded to assess the antibiofilm activity of the gold+graphene complex or mixture (aqueous phase of the cream).

The testing performed comprises biofilm formation inhibition assessment, using concentrations lower than the MIC of the gold-graphene-lipoic acid mixture (gold nanoparticles 0.05 mg/ml + graphene 0.05 mg/ml + lipoic acid 0.0005 mg/ml) and the ability to disrupt a 24h-preformed biofilm at concentrations equal to (in case of S. *agalactiae)* or higher than (in case of E.*coli* and E. *faecalis)* the MIC.

The method used is a Micromethod in a 96-well plate with crystal violet staining of the adhered biomass and measurement of the optical density of sample wells, and comparison with growth control wells (untreated) and negative control wells (culture medium and sample at different concentrations only, but without microbial inoculum).

Obtained results are reported in Tables 6 and 7.

Biofilm formation inhibition activity was performed at two different sub-MIC concentrations: 2% and 1% v/v (values lower than the MIC obtained toward planktonic strains). Biofilm formation inhibition is reported in terms of inhibition percent compared to untreated growth control.

Table 6. **Biofilm formation inhibition at sub-MIC concentrations**

| | **Biofilm formation inhibition percent** | |
|---|---|---|
| Gold-graphene-lipoic acid mixture (0.05 mg/ml + 0.05 mg/ml + 0.0005 mg/ml) as indicated above | **2% v/v** | **1%v/v** |
| E. *coli* ATCC 25922 | 39.3 ± 0.01 | 17.4 ± 0.9 |
| E. *faecalis* ATCC 29212 | 41.5 ± 0.4 | 36.3 ± 1.9 |
| S. *agalactiae* ATCC 13813 | 48.7± 4.0 | 32.6± 0.9 |

Mean values ± standard deviation of two independent tests in triplicate

The gold-graphene-lipoic acid mixture (gold nanoparticles 0.05 mg/ml + graphene 0.05 mg/ml + lipoic acid 0.0005 mg/ml) interferes with the forming of the biofilm of *E. coli* ATCC 25922, *E. faecalis* ATCC 29212 and S. *agalactiae* ATCC 13813 strains, particularly at the concentration of 2% v/v (lower than the MIC against free-living strains).

The activity disrupting a 24h-preformed biofilm was performed at a concentration of 50% v/v, equal to the MIC in the case of S. *agalactiae* or higher than the MIC (10% and 5% v/v) in the case of *E. coli* and *E. faecalis.*

Table 7. **Activity disrupting a preformed biofilm (24h) at concentrations equal to or higher than the MIC**

| Activity disrupting the preformed biofilm (24h) Inhibition percent | | | |
|---|---|---|---|
| Gold-graphene-lipoic acid mixture (0.05 mg/ml + 0.05 mg/ml + 0.0005 mg/ml) as indicated above | **50% v/v** | **10% v/v** | **5% v/v** |
| *E. coli* ATCC 25922 | nt | 78±3.5 | 69.3±0.5 |
| *E. faecalis* ATCC 29212 | nt | 38 ± 2.9 | 29.1±0.9 |
| S. *agalactiae* ATCC 13813 | 36.2± 0.9 | nt | nt |

Mean values ± standard deviation of two independent experiments in triplicate; nt= not tested

Conclusive remarks:

The gold-graphene-lipoic acid mixture (gold nanoparticles 0.05 mg/ml + graphene 0.05 mg/ml + lipoic acid 0.0005 mg/ml) (aqueous phase) interferes with the formation of the biofilm of *E. coli* ATCC 25922, *E. faecalis* ATCC 29212 and S. *agalactiae* ATCC 13813 reference strains, particularly at a concentration of 2% v/v (lower than the MIC against the free-living strains).

The gold-graphene-lipoic acid mixture(gold nanoparticles 0.05 mg/ml + graphene 0.05 mg/ml + lipoic acid 0.0005 mg/ml) at concentrations higher than the MIC (10% and 5% v/v) has an interesting activity disrupting a 24h-preformed biofilm, particularly toward E. *coli* ATCC 25922.

## EXAMPLE 4

### FIC CALCULATION OF GOLD+GRAPHENE COMPLEX VS GOLD NANOPARTICLES AND GRAPHENE NANOPARTICLES

In order to determine the synergistic effect between the active agents gold and graphene, joined to form the gold-graphene complex, the FIC *(Fractional Inhibitory Concentration)* index was used, which measures the interaction between two or more active agents, joined to form a complex, starting from the MIC *(Minimal Inhibitory Concentration)* values of the same active agents considered individually or in combination, according to the following formula:

$$\underline{FIC = Active\ A\ FIC + Active\ B\ FIC}$$

$$Active\ A\ FIC = MIC\ of\ Active\ A\ in\ combination\ /\ MIC\ of\ Active\ A\ alone$$

$$Active\ B\ FIC = MIC\ of\ Agent\ B\ in\ combination\ /\ MIC\ Agent\ B\ alone$$

### INTERPRETATION OF RESULTS

| SYNERGISM | | $\Sigma$ **FIC < 1.0** |
|---|---|---|
| | MARKED | $\Sigma$ FIC < 0.5 |
| | WEAK | $0.5 < \Sigma$ FIC < 1.0 |
| ADDITIVE SYNERGISM | | $\Sigma$ **FIC = 1.0** |

(continued)

| SYNERGISM | | Σ FIC < 1.0 |
|---|---|---|
| | MARKED | Σ FIC < 0.5 |
| | WEAK | 0.5 < Σ FIC < 1.0 |
| SUBADDITIVE SYNERGISM | | 1.0 < Σ FIC < 2.0 |
| INDIFFERENCE | | Σ FIC = 2.0 |
| ANTAGONISM | | Σ FIC > 2.0 |

**Antibacterial and antifungal activity of the aqueous formulation of the gold+graphene complex**

| MIC in % v/v or (mg/ml) | | | | | |
|---|---|---|---|---|---|
| | *E. coli* ATCC 25922 | *E. faecalis* ATCC 29212 | *S. agalactiae* ATCC 13813 | *C. albicans* ATCC 10536 | *C. krusei* ATCC 14243 |
| Gold+graphene compound (0.05mg/ml+0.05m g/ml) 50μg/ml+50 μg/ml | 3,1% (1.5μg/ml+1.5μg/ml) | 3,1% (1.5μg/ml+1.5μg/ml) | 50% (25 μg /ml+25 μg /ml) | 12,5% (6.2μg/ml+6,2μg/ml) | 50% (25 μg /ml+25 μg /ml) |
| Gold nanoparticles (0.06mg/ml) | 50% (0.03mg/ml) | 50% (0.03mg/ml) | 50% (0.03mg/ml) | 50% (0.03mg/ml) | 50% (0.03mg/ml) |
| Graphene (2mg/ml) | 25% (0.5mg/ml) | 25% (0.5mg/ml) | 50% (1mg/ml) | 50% (1mg/ml) | 50% (1mg/ml) |

**FIC calculation**

| *Escherichia Coli*<br>FIC =(3.1 : 30) + (3.1 : 500)= 0.10 + 0.0062= 0.10 | MARKED SYNERGISM |
|---|---|
| *Enterococcus Faecalis*<br>FIC = (3.1 : 30) + (3.1 : 500)= 0.10 + 0.0062= 0.10 | MARKED SYNERGISM |
| *Streptococcus Agalactiae*<br>FIC = (50 : 30) + (50 : 1000)= 1.66 + 0.05= 1.71 | SUBADDITIVE SYNERGISM |
| *Candida Albicans*<br>FIC= (12.5 : 30) + (12.5 : 1000) = 0.41 + 0.01=0.42 | WEAK SYNERGISM |
| *Candida Krusei*<br>FIC= (50 : 30) + (50: 1000) = 1.66 + 0.05= 1.71 | SUBADDITIVE SYNERGISM |

FIC index analysis reveals a synergism of the gold-graphene complex vs the single active agents gold and graphene considered individually; specifically, a Marked Synergism toward *Escherichia Coli* and *Enterococcus Faecalis;* a Weak Synergism toward *Candida Albicans* and a subadditive Synergism toward *Streptococcus Agalactiae* and *Candida Krusei* are highlighted. In all cases, markedly in additive synergism ones, but also in weak and subadditive synergism cases, by analyzing the MICs a superiority of the gold-graphene complex is detected compared to the individually considered active agents, that can be highlighted from the concentrations expressed in the Table. The calculation was performed according to what described in Singh AP, Prabha V, Rishi P Value addition in the efficacy of conventional antibiotics by Nisin against Salmonella. PLoS One. 2013 Oct 8;8(10):e76844.

STUDY UNDER WAY

An additional validation study of the effectiveness of the gold-graphene complex according to the present description, by means of *in vivo* tests on animal model for vaginal candidiasis, is under way.

Animals are treated in compliance with European directives and national regulations on the management of laboratory animals.

The selected animals are Wistar rats or adult female mice.

Candidiasis is induced in the animals, followed by validation thereof by means of analysis of the state of candidiasis (fungal count).

There follow

One or more applications of the gold-graphene complex as defined herein to the animal models at periodic time intervals,

Assessment of infection reduction after a single application and after multiple applications over one week,

The animals are split into four (4) groups, each comprised of six (6) animals, differentiated as follows:

- Group 1, healthy control: no dosage form administered.
- Group 2, control with candidiasis: no dosage form administered.
- Group 3, with candidiasis: single administration and analysis of the state of candidiasis at a preset time.
- Group 4, with candidiasis: multiple administration (e.g., once a day for 6 days) and analysis of the state of candidiasis at a preset time.

Validation of the effectiveness of the formulation containing the gold-graphene complex

**Claims**

1. A mixture comprising:

   - gold particles, and
   - graphene particles or a derivative thereof or mixtures thereof,
   and a linker binding said gold particles to said graphene particles or derivative thereof or mixtures thereof,
   wherein said linker is alpha lipoic acid,
   for use in the treatment and/or prevention of urogenital tract infections,
   wherein said derivative is graphene oxide.

2. The mixture for use according to claim 1, further comprising an aqueous phase with an acid pH, said phase comprising at least one organic acid.

3. The mixture for use according to claim 2, wherein said pH is from 3.0 to 5.5 or wherein said pH is from 3.5 to 5 or wherein said pH is about 4.5.

4. The mixture for use according to anyone of claims 2 or 3, wherein said acid is an organic acid with from one to eight carbon atoms.

5. The mixture for use according to claim 4, wherein said organic acid is lactic acid.

6. The mixture for use according to anyone of claims 1 to 5, wherein said gold particles and said graphene particles or derivative thereof have sizes from 1 nm to 2 $\mu$m, or from 20 nm to 1 $\mu$m and/or, wherein the weight ratio between said gold particles and said graphene particles or derivative thereof or mixtures thereof is from 1:1 to 1:100, or from 1:1 to 1:40, or is 1:1; or
   wherein the weight ratio between said graphene particles or derivative thereof or mixtures thereof and said gold particles is from 1:1 to 1:100, or from 1:1 to 1:40, and/or wherein the concentration of said gold particles is from 0.00001% to 0.2% w/w, or from 0.00001 to 0.1 w/w, or from 0.00001 to 0.004% w/w, and the concentration of said graphene particles or derivative thereof or mixtures thereof, is from 0.00001% to 0.2% w/w, or from 0.00001 to 0.1 w/w, or from 0.00001 to 0.004% w/w.

7. The mixture for use according to anyone of claims 1 to 6, wherein said alpha lipoic acid has a concentration ranging from 0.0000001 to 0.1% w/w, or from 0.0000001 to 0.00001% w/w.

8. A mixture comprising:

   gold particles,
   graphene oxide particles, and
   lactic acid
   and a linker binding said gold particles to said graphene oxide particles wherein said linker is alpha lipoic acid wherein said mixture has a pH of between 3.5 and 7, or wherein said mixture has a pH of between 4 and 6, or wherein said mixture has a pH of 4.5.

9. The mixture according to claim 8 wherein said gold particles have sizes from 1 nm to 2 $\mu$m, or from 20 nm to 1 $\mu$m and said graphene oxide particles have sizes from 1 nm to 2 $\mu$m, or from 20 nm to 1 $\mu$m and/or, wherein the weight ratio between said gold particles and said graphene oxide particles is from 1:1 to 1:40, or is 1:1; or wherein the weight ratio between said graphene oxide particles and said gold particles is from 1:1 to 1:40 and/or, wherein the concentration of said gold particles is from 0.00001% to 0.2% w/w, or from 0.00001 to 0.1% w/w, or from 0.00001 to 0.004% w/w, or from 0.0001 to 0.004% w/w, or from 0.001 to 0.1% w/w and the concentration of said graphene oxide particles is from 0.00001% to 0.2% w/w, or from 0.00001 to 0.1% w/w or from 0.00001 to 0.004% w/w, or from 0.0001 to 0.004% w/w or from 0.001 to 0.1% w/w and/or, wherein said alpha lipoic acid has a concentration ranging from 0.0000001 to 0.1% w/w, or from 0.0000001 to 0.00001% w/w.

10. The mixture according to claim 8 or 9, wherein said mixture comprises, for 100 g of final formulation:

    0.001 g of gold particles,
    0.001 g of graphene oxide particles,
    0.9 g of lactic acid, and
    0.00001 g of alpha lipoic acid.

11. A pharmaceutical composition comprising the mixture according to anyone of claims 1 to 10, and at least one pharmaceutically acceptable excipient.

12. The pharmaceutical composition according to claim 11, in liquid, solid or semi-solid form, in the form of cream, gel, cream-gel, emulgel, softgel, foam, emulsion, sprayable emulsion, suppository.

13. The pharmaceutical composition according to claim 12, wherein said cream composition is an O/W cream consisting of:

    0.001 g of gold particles
    0.001 g of graphene oxide particles
    0.00001 g of alpha lipoic acid
    0.5 g of lactic acid
    30 g of liquid paraffin
    10 g of cetearyl alcohol
    4 g of PEG 1500
    1g of Tween 80
    Water q.s. to 100 g.

14. The mixture according to anyone of claims 8 to 10, or the composition according to anyone of claims 11 to 13 for use as a medicament.

15. The mixture according to anyone of claims 8 to 10, or the composition according to anyone of claims 11 to 13 for use in the treatment and/or prevention of urogenital tract infections.

16. The mixture or the composition for use according to claim 15, or the mixture for use according to anyone of claims 1 to 7 wherein said infections are bacterial and/or fungal and/or protozoan and/or mycoplasma infections.

17. The mixture or the composition for use according to claim 16, wherein said bacterial infections are caused by gram-negative, gram-positive and/or gram-variable bacteria; and in which said fungal infections are caused by a fungus of the genus Candida.

**18.** The mixture or the composition for use according to anyone of claims 14 to 17 wherein said use is a topical use.

**19.** The mixture or the composition for use according to claim 18, wherein said topical use occurs by applying said mixture or composition externally, internally, on the skin, on the mucosa, in the vagina, on the vulva or on the penis.

**20.** A kit, vaginal capsule, vaginal tablet, vaginal softgel, ovum, bandage, plaster, wadding, spray, vaginal film, probe comprising the mixture as defined in anyone of claims 1 to 10 or the pharmaceutical composition according to anyone of claims 11 to 13.

**Patentansprüche**

**1.** Mischung, umfassend:

- Goldpartikel und
- Graphenpartikel oder ein Derivat davon oder Mischungen davon,

und einen Linker, der die Goldpartikel an die Graphenpartikel oder ein Derivat davon oder Mischungen davon bindet, wobei der Linker Alpha-Liponsäure ist, zur Verwendung bei der Behandlung und/oder Vorbeugung von Infektionen des Urogenitaltrakts, wobei das Derivat Graphenoxid ist.

**2.** Mischung zur Verwendung nach Anspruch 1, ferner umfassend eine wässrige Phase mit einem sauren pH-Wert, die Phase umfassend mindestens eine organische Säure.

**3.** Mischung zur Verwendung nach Anspruch 2, wobei der pH-Wert von 3,0 bis 5,5 beträgt oder wobei der pH-Wert von 3,5 bis 5 beträgt oder wobei der pH-Wert etwa 4,5 beträgt.

**4.** Mischung zur Verwendung nach einem der Ansprüche 2 oder 3, wobei die Säure eine organische Säure mit von ein bis acht Kohlenstoffatomen ist.

**5.** Mischung zur Verwendung nach Anspruch 4, wobei die organische Säure Milchsäure ist.

**6.** Die Mischung zur Verwendung nach einem der Ansprüche 1 bis 5, wobei die Goldpartikel und die Graphenpartikel oder einem Derivat davon Größen von 1 nm bis 2 $\mu$m oder von 20 nm bis 1 $\mu$m aufweisen und/oder wobei das Gewichtsverhältnis zwischen den Goldpartikeln und den Graphenpartikeln oder einem Derivat davon oder Mischungen davon von 1 : 1 bis 1 : 100 oder 1 : 1 bis 1 : 40 oder 1 : 1 beträgt; oder wobei das Gewichtsverhältnis zwischen den Graphenpartikeln oder einem Derivat davon oder Mischungen davon und den Goldpartikeln von 1 : 1 bis 1 : 100 oder von 1 : 1 bis 1 : 40 beträgt und/oder wobei die Konzentration der Goldpartikel von 0,00001 Gew.-% bis 0,2 Gew.-% oder von 0,00001 bis 0,1 Gew.-% oder von 0,00001 bis 0,004 Gew.-% beträgt und die Konzentration der Graphenpartikel oder einem Derivat davon oder Mischungen davon von 0,00001 Gew.-% bis 0,2 Gew.-% oder von 0,00001 bis 0,1 Gew.-% oder von 0,00001 bis 0,004 Gew.-% beträgt.

**7.** Mischung zur Verwendung nach einem der Ansprüche 1 bis 6, wobei die Alpha-Liponsäure eine Konzentration in dem Bereich von 0,0000001 bis 0,1 Gew.-% oder von 0,0000001 bis 0,00001 Gew.-% aufweist.

**8.** Mischung, umfassend:

Goldpartikel,
Graphenoxidpartikel und
Milchsäure
und ein Linker, der die Goldpartikel an die Graphenoxidpartikel bindet, wobei der Linker Alpha-Liponsäure ist, wobei die Mischung einen pH-Wert zwischen 3,5 und 7 aufweist, oder wobei die Mischung einen pH-Wert zwischen 4 und 6 aufweist, oder wobei die Mischung einen pH-Wert von 4,5 aufweist.

**9.** Mischung nach Anspruch 8, wobei die Goldpartikel Größen von 1 nm bis 2 $\mu$m oder von 20 nm bis 1 $\mu$m aufweisen und die Graphenoxidpartikel Größen von 1 nm bis 2 $\mu$m oder von 20 nm bis 1 $\mu$m aufweisen und/oder wobei das Gewichtsverhältnis zwischen den Goldpartikeln und den Graphenoxidpartikeln von 1 : 1 bis 1 : 40 oder 1 : 1 beträgt;

oder wobei das Gewichtsverhältnis zwischen den Graphenoxidpartikeln und den Goldpartikeln von 1 : 1 bis 1 : 40 beträgt und/oder wobei die Konzentration der Goldpartikel von 0,00001 Gew.-% bis 0,2 Gew.-% oder von 0,00001 bis 0,1 Gew.-% oder von 0,00001 bis 0,004 Gew.-% oder von 0,0001 bis 0,004 Gew.-% oder von 0,001 bis 0,1 Gew.-% beträgt und die Konzentration der Graphenoxidpartikel von 0,00001 Gew.-% bis 0,2 Gew.-% oder von 0,00001 bis 0,1 Gew.-% oder von 0,00001 bis 0,004 Gew.-% oder von 0,0001 bis 0,004 Gew.-% oder von 0,001 bis 0,1 Gew.-% beträgt und/oder wobei die Alpha-Liponsäure eine Konzentration in dem Bereich von 0,0000001 bis 0,1 Gew.-% oder von 0,0000001 bis 0,00001 Gew.-% aufweist.

10. Mischung nach Anspruch 8 oder 9, wobei die Mischung pro 100 g der Endformulierung umfasst:

   zu 0,001 g Goldpartikel,
   zu 0,001 g Graphenoxidpartikel,
   zu 0,9 g Milchsäure und
   zu 0,00001 g Alpha-Liponsäure.

11. Pharmazeutische Zusammensetzung, umfassend die Mischung nach einem der Ansprüche 1 bis 10 und mindestens einen pharmazeutisch verträglichen Träger.

12. Pharmazeutische Zusammensetzung nach Anspruch 11 in flüssiger, fester oder halbfester Form, in Form einer Creme, eines Gels, eines Creme-Gels, eines Emulgels, einer Weichkapsel, eines Schaums, einer Emulsion, einer sprühbaren Emulsion oder eines Zäpfchens.

13. Pharmazeutische Zusammensetzung nach Anspruch 12, wobei die Cremezusammensetzung eine O/W-Creme ist, bestehend aus:

   zu 0,001 g Goldpartikeln
   zu 0,001 g Graphenoxidpartikeln
   zu 0,00001 g Alpha-Liponsäure
   zu 0,5 g Milchsäure
   zu 30 g flüssigem Paraffin
   zu 10 g Cetearylalkohol
   zu 4 g PEG 1500
   zu 1 g Tween 80
   Wasser q.s. auf 100 g.

14. Mischung nach einem der Ansprüche 8 bis 10 oder die Zusammensetzung nach einem der Ansprüche 11 bis 13 zur Verwendung als Medikament.

15. Mischung nach einem der Ansprüche 8 bis 10 oder die Zusammensetzung nach einem der Ansprüche 11 bis 13 zur Verwendung bei der Behandlung und/oder Vorbeugung von Infektionen des Urogenitaltrakts.

16. Mischung oder die Zusammensetzung zur Verwendung nach Anspruch 15 oder die Mischung zur Verwendung nach einem der Ansprüche 1 bis 7, wobei die Infektionen bakterielle und/oder Pilz- und/oder Protozoen- und/oder Mykoplasmen-Infektionen sind.

17. Mischung oder die Zusammensetzung zur Verwendung nach Anspruch 16, wobei die bakteriellen Infektionen durch gramnegative, grampositive und/oder gramvariable Bakterien verursacht werden; und wobei die Pilzinfektionen durch einen Pilz der Gattung Candida verursacht werden.

18. Mischung oder Zusammensetzung zur Verwendung nach einem der Ansprüche 14 bis 17, wobei es sich bei der Verwendung um eine topische Verwendung handelt.

19. Die Mischung oder die Zusammensetzung zur Verwendung nach Anspruch 18, wobei die topische Verwendung durch Auftragen der Mischung oder Zusammensetzung äußerlich, innerlich, auf die Haut, auf die Schleimhaut, in der Vagina, auf die Vulva oder auf den Penis erfolgt.

20. Kit, eine Vaginalkapsel, eine Vaginaltablette, eine Vaginalweichgelkapsel, ein Ovum, ein Verband, ein Pflaster, eine Watte, ein Spray, ein Vaginalfilm oder eine Sonde, umfassend die Mischung nach einem der Ansprüche 1 bis 10 oder die pharmazeutische Zusammensetzung nach einem der Ansprüche 11 bis 13.

**Revendications**

1. Mélange comprenant :

   - des particules d'or, et
   - des particules de graphène ou leurs dérivés ou leurs mélanges,

     et un lieur liant lesdites particules d'or auxdites particules de graphène ou à leurs dérivés ou à leurs mélanges, dans lequel ledit lieur est de l'acide alpha-lipoïque,
     à utiliser dans le traitement et/ou la prévention d'infections urinaires, dans lequel ledit dérivé est de l'oxyde de graphène.

2. Mélange à utiliser selon la revendication 1, comprenant en outre une phase aqueuse à pH acide, ladite phase comprenant au moins un acide organique.

3. Mélange à utiliser selon la revendication 2, dans lequel le pH est compris entre 3,0 et 5,5 ou dans lequel ledit pH est de 3,5 et 5 ou dans lequel ledit pH est d'environ 4,5.

4. Mélange à utiliser selon l'une quelconque des revendications 2 ou 3, dans lequel ledit acide est un acide organique ayant de un à huit atomes de carbone.

5. Mélange à utiliser selon la revendication 4, dans lequel ledit acide organique est de l'acide lactique.

6. Mélange à utiliser selon l'une quelconque des revendications 1 à 5, dans lequel lesdites particules d'or et lesdites particules de graphène ou leurs dérivés ont des tailles allant de 1 nm à 2 $\mu$m, ou de 20 nm à 1 $\mu$m et/ou, dans lequel le rapport de poids entre lesdites particules d'or et lesdites particules de graphène ou leurs dérivés ou leurs mélanges est de 1:1 à 1:100, ou de 1:1 à 1:40, ou est de 1:1 ; ou
   dans lequel le rapport de poids entre lesdites particules de graphène ou leurs dérivés ou leurs mélanges et lesdites particules d'or est de 1:1 à 1:100, ou de 1:1 à 1:40, et/ou dans lequel la concentration desdites particules d'or est de 0,00001 % à 0.2 % p/p, ou de 0,00001 à 0,1 p/p, ou de 0,00001 à 0,004 % p/p, et la concentration desdites particules de graphène ou de leurs dérivés, ou de leurs mélanges, est de 0,00001 % à 0,2 % p/p, ou de 0,00001 à 0,1 p/p, ou de 0,00001 à 0,004 % p/p.

7. Mélange à utiliser selon l'une quelconque des revendications 1 à 6, dans lequel ledit acide alpha-lipoïque a une concentration allant de 0,0000001 à 0,1 % p/p, ou de 0,0000001 à 0,00001 % p/p.

8. Mélange comprenant :

   des particules d'or,
   des particules d'oxyde de graphène, et
   de l'acide lactique
   et un lieur liant lesdites particules d'or auxdites particules d'oxyde de graphène, dans lequel ledit lieur est de l'acide alpha-lipoïque
   dans lequel ledit mélange a un pH compris entre 3,5 et 7, ou dans lequel ledit mélange a un pH compris entre 4 et 6, ou dans lequel ledit mélange a un pH de 4,5.

9. Mélange selon la revendication 8, dans lequel lesdites particules d'or ont des tailles de 1 nm à 2 $\mu$m, ou de 20 nm à 1 $\mu$m et lesdites particules d'oxyde de graphène ont des tailles de 1 nm à 2 $\mu$m, ou de 20 nm à 1 $\mu$m et/ou, dans lequel le rapport de poids entre lesdites particules d'or et lesdites particules d'oxyde de graphène est de 1:1 à 1:40, ou est 1:1 ; ou dans lequel le rapport de poids entre lesdites particules d'oxyde de graphène et lesdites particules d'or est de 1:1 à 1:40 et/ou, dans lequel la concentration desdites particules d'or est de 0,00001 % à 0,2 % p/p, ou de 0,00001 à 0,1 % p/p, ou de 0,00001 à 0,004 % p/p, ou de 0,0001 à 0,004 % p/p, ou de 0,001 à 0,1 % p/p et la concentration desdites particules d'oxyde de graphène est de 0,00001 % à 0,2 % p/p, ou de 0,00001 à 0,1 % p/p ou de 0,00001 à 0,004 % p/p, ou de 0,0001 à 0,004 % p/p ou de 0,001 à 0,1 % p/p et/ou, dans lequel ledit acide alpha-lipoïque a une concentration allant de 0,0000001 à 0,1 % p/p, ou de 0,0000001 à 0,00001 % p/p.

**10.** Mélange selon la revendication 8 ou 9, dans lequel ledit mélange comprend, pour 100 g de formulation finale :

> 0,001 g de particules d'or,
> 0,001 g de particules d'oxyde de graphène,
> 0,9 g d'acide lactique, et
> 0,00001 g d'acide alpha lipoïque.

**11.** Composition pharmaceutique comprenant le mélange selon l'une quelconque des revendications 1 à 10, et au moins un excipient pharmaceutiquement acceptable.

**12.** Composition pharmaceutique selon la revendication 11, sous forme liquide, solide ou semi-solide, sous forme de crème, de gel, de gel-crème, d'émulgel, de gel mou, de mousse, d'émulsion, d'émulsion pulvérisable, de suppositoire.

**13.** Composition pharmaceutique selon la revendication 12, dans laquelle ladite composition de crème est une crème H/E constituée de :

> 0,001 g de particules d'or
> 0,001 g de particules d'oxyde de graphène
> 0,00001 g d'acide alpha-lipoïque
> 0,5 g d'acide lactique
> 30 g de paraffine liquide
> 10 g d'alcool cétéarylique
> 4 g de PEG 1500
> 1 g de Tween 80
> Eau q.s. à 100 g.

**14.** Mélange selon l'une quelconque des revendications 8 à 10, ou la composition selon l'une quelconque des revendications 11 à 13, à utiliser en tant que médicament.

**15.** Mélange selon l'une quelconque des revendications 8 à 10, ou la composition selon l'une quelconque des revendications 11 à 13, à utiliser le traitement et/ou la prévention d'infections urinaires.

**16.** Mélange ou composition à utiliser selon la revendication 15, ou mélange à utiliser selon l'une quelconque des revendications 1 à 7, dans lequel lesdites infections sont des infections bactériennes et/ou fongiques et/ou protozoaires et/ou mycoplasmes.

**17.** Mélange ou composition à utiliser selon la revendication 16, dans lequel lesdites infections bactériennes sont causées par des bactéries gram-négatives, gram-positives et/ou gram-variables ; et dans lequel lesdites infections fongiques sont causées par un champignon du genre Candida.

**18.** Mélange ou composition à utiliser selon l'une quelconque des revendications 14 à 17, dans lequel ladite utilisation est une utilisation topique.

**19.** Mélange ou composition à utiliser selon la revendication 18, dans lequel l'utilisation topique se fait par application dudit mélange ou de la composition en externe, en interne, sur la peau, sur la muqueuse, dans le vagin, sur la vulve ou sur le pénis.

**20.** Trousse, capsule vaginale, comprimé vaginal, capsule molle vaginale, ovule, pansement, plâtre, ouate, vaporisateur, film vaginal, sonde comprenant le mélange tel que défini dans l'une quelconque des revendications 1 à 10 ou composition pharmaceutique selon l'une quelconque des revendications 11 à 13.

Sample Name: GNPs IOI 1
SOP Name: Roberto.sop
File Name: Roberto.dts                              Dispersant Name: Water
Record Number: 15                                   Dispersant RI: 1,330
Material RI: 1,59                                   Viscosity (cP): 0,8872
Material Absorption: 0,010                          Measurement Date and Time:          22/01/2019 1

Temperature (°C): 25,0                              Duration Used (s): 60
Count Rate (kcps): 146,6                            Measurement Position (mm): 3,00
Cell Description: Disposable micro cuvette (40μl)   Attenuator: 6

|  |  | Size (d.nm): | % Intensity: | St Dev (d.nm): |
|---|---|---|---|---|
| Z-Average (d.nm): 28,11 | Peak 1: | 34,78 | 100,0 | 13,06 |
| Pdl: 0,198 | Peak 2: | 0,000 | 0,0 | 0,000 |
| Intercept: 0,874 | Peak 3: | 0,000 | 0,0 | 0,000 |
| Result quality : Good |  |  |  |  |

FIG 1

| | | Size (d.nm): | % Intensity: | St Dev (d.nm): |
|---|---|---|---|---|
| Sample Name: | Graphene Oxide Nanocolloids 1 | | | |
| SOP Name: | Roberto.sop | | | |
| File Name: | Roberto.dts | Dispersant Name: | Water | |
| Record Number: | 16 | Dispersant RI: | 1,330 | |
| Material RI: | 1,59 | Viscosity (cP): | 0,8872 | |
| Material Absorption: | 0,010 | Measurement Date and Time: | / | 22/01/2019 11:21:9 |

| | | | | |
|---|---|---|---|---|
| Temperature (°C): | 25,0 | Duration Used (s): | 60 | |
| Count Rate (kcps): | 334,6 | Measurement Position (mm): | 3,00 | |
| Cell Description: | Disposable micro cuvette (40µl) | Attenuator: | 10 | |

| | | | Size (d.nm): | % Intensity: | St Dev (d.nm): |
|---|---|---|---|---|---|
| Z-Average (d.nm): | 593,5 | Peak 1: | 561,4 | 100,0 | 110,1 |
| Pdi: | 0,289 | Peak 2: | 0,000 | 0,0 | 0,000 |
| Intercept: | 0,856 | Peak 3: | 0,000 | 0,0 | 0,000 |
| Result quality : | Refer to quality report | | | | |

Fig.2

Fig 3

Sample Name: Complesso OG pH 4.5 ac.latt. 1
SOP Name: Roberto.sop
File Name: Roberto.dts
Record Number: 18
Material RI: 1,59
Material Absorbtion: 0,010

Dispersant Name: Water
Dispersant RI: 1,330
Viscosity (cP): 0,8872
Measurement Date and Time: 22/01/ 2019 - 12

Temperature (°C): 25,0
Count Rate (kcps): 230,8
Cell Description: Disposable micro cuvette (40μl)

Duration Used (s): 70
Measurement Position (mm): 3,00
Attenuator: 6

| | | Size (d.nm): | % Intensity: | St Dev (d.nm): |
|---|---|---|---|---|
| Z-Average (d.nm): 62,04 | Peak 1: | 616,3 | 52,5 | 251,0 |
| Pdi: 0,629 | Peak 2: | 35,95 | 47,5 | 10,51 |
| Intercept: 0,913 | Peak 3: | 0,000 | 0,0 | 0,000 |

Result quality : Refer to quality report

Fig. 4

Sample Name: OG ph 4.5 + Ac lipoico 200uL 1
SOP Name: Roberto.sop
File Name: Roberto.dts      Dispersant Name: Water
Record Number: 28      Dispersant RI: 1,330
Material RI: 1,59      Viscosity (cP): 0,8872
Material Absorption: 0,010   Measurement Date and Time: 23/01/2019 11

Temperature (°C): 25,0    Duration Used (s): 80
Count Rate (kcps): 101,0   Measurement Position (mm): 3,00
Cell Description: Disposable micro cuvette (40µl)  Attenuator: 5

|  |  | Size (d.nm): | % Intensity: | St Dev (d.nm): |
|---|---|---|---|---|
| Z-Average (d.nm): 227,1 | Peak 1: | 88,27 | 45,3 | 24,95 |
| Pdl: 0,641 | Peak 2: | 776,3 | 40,9 | 171,4 |
| Intercept: 0,912 | Peak 3: | 30,15 | 13,8 | 7,306 |

Result quality : Refer to quality report

**Size Distribution by Intensity**

Record 28: OG ph 4.5 + Ac lipoico 200uL 1

Fig. 5

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **K TURCHENIUK et al.** Plasmonic photothermal destruction of uropathogenic E.coli with reduced graphene oxide and core/shell nanocomposites of gold nanorods/reduced graphene oxide. *JOURNAL OF MATERIALS CHEMISTRY B,* 19 November 2014, vol. 3 (3), 375-386
- **K TURCHENIUK et al.** Gold-graphene nanocomposites for sensing and biomedical applications. *JOURNAL OF MATERIALS CHEMISTRY B,* 01 January 2015, vol. 3 (21), 4301-4324
- **M. LOTYA et al.** *Nanotechnology,* 2013, vol. 24, 265703
- **SINGH AP ; PRABHA V ; RISHI P.** Value addition in the efficacy of conventional antibiotics by Nisin against Salmonella. *PLoS One.,* 08 October 2013, vol. 8 (10), e76844